(19) Europäisches Patentamt / European Patent Office / Office européen des brevets

(11) **EP 3 289 975 A1**

(12) **EUROPEAN PATENT APPLICATION**

(43) Date of publication:
**07.03.2018 Bulletin 2018/10**

(51) Int Cl.:
**A61B 5/11** (2006.01)  **G08B 21/04** (2006.01)
**G16H 40/67** (2018.01)  **A61B 8/08** (2006.01)

(21) Application number: **17189288.8**

(22) Date of filing: **04.09.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **04.09.2016 US 201662383443 P**

(71) Applicant: **Essence Smartcare Ltd**
**4672530 Herzlia Pituach (IL)**

(72) Inventor: **REGEV, Nir**
**4482000 Barkan (IL)**

(74) Representative: **Kramer, Dani et al**
**Mathys & Squire LLP**
**The Shard**
**32 London Bridge Street**
**London SE1 9SG (GB)**

(54) **DETECTING FALLS AND NEAR FALLS BY ULTRASOUND**

(57) There is provided a system (200) for non-contact computation of an indication of at least one of a fall and a near-fall of a target individual (206) based on ultrasound transmission, comprising: at least one ultrasound transceiver (202) configured for generating an ultrasound transmission (204) and receiving a reflection (208) of the ultrasound transmission, and at least one processor (212) in communication with the ultrasound transceiver, the at least one processor configured for: activating (102) the at least one ultrasound transceiver for generating the ultrasound transmission, and for receiving (104) the reflection of the ultrasound transmission, performing (110) a spectrogram analysis of the received reflection of the ultrasound transmission to compute a plurality of features, and computing (116) an indication of at least one of: a fall of a target individual, and a near-fall of the target individual, by a statistical classifier classifying the plurality of features.

FIG. 2

## Description

### RELATED APPLICATION

This application claims the benefit of priority under 35 USC § 119(e) of U.S. Provisional Patent Application No. 62/383,443 filed September 4, 2016, the contents of which are incorporated herein by reference in their entirety.

### BACKGROUND

The present invention, in some embodiments thereof, relates to fall detection and, more specifically, but not exclusively, to systems and methods for non-contact detection of a fall of a target individual.

The number and age of senior citizens is constantly increasing. Many of the senior citizens live at home alone, with a spouse, or with hired help. Senior citizens are especially prone to falling, which may result in severe complications. Elderly people who are independent and function satisfactorily in their daily life, are often hospitalized after a fall, and often never regain their health.

Systems for preventing or detecting a fall are sought.

### SUMMARY

Aspects and embodiments of the present invention are set out in the appended claims. These and other aspects and embodiments of the invention are also described herein.

According to a first aspect, a system for non-contact computation of an indication of at least one of a fall and a near-fall of a target individual based on ultrasound transmission, comprising: at least one ultrasound transceiver configured for generating an ultrasound transmission and receiving a reflection of the ultrasound transmission, and at least one processor in communication with the ultrasound transceiver, the at least one processor configured for: activating the at least one ultrasound transceiver for generating the ultrasound transmission, and for receiving the reflection of the ultrasound transmission, performing a spectrogram analysis of the received reflection of the ultrasound transmission to compute a plurality of features, and computing an indication of at least one of: a fall of a target individual, and a near-fall of the target individual, by a statistical classifier classifying the plurality of features.

According to a second aspect, a method of non-contact computation of an indication of at least one of a fall and a near-fall of a target individual based on ultrasound transmission, comprising: generating an ultrasound transmission by at least one ultrasound transceiver, receiving a reflection of the ultrasound transmission by at least one ultrasound transceiver, performing a spectrogram analysis of the received reflection of the ultrasound transmission to compute a plurality of features, and computing an indication of at least one of: a fall of a target individual, and a near-fall of the target individual, by a statistical classifier classifying the plurality of features.

According to a third aspect, a computer program product for non-contact computation of an indication of at least one of a fall and a near-fall of a target individual based on ultrasound transmission, comprising: a non-transitory memory having stored there a code for execution by at least one hardware processor of a computing device, the code comprising: instructions for generating an ultrasound transmission by at least one ultrasound transceiver, instructions for receiving a reflection of the ultrasound transmission by at least one ultrasound transceiver, instructions for performing a spectrogram analysis of the received reflection of the ultrasound transmission to compute a plurality of features, and instructions for computing an indication of at least one of: a fall of a target individual, and a near-fall of the target individual, by a statistical classifier classifying the plurality of features.

The systems, apparatus, methods, and/or code instruction described herein address the technical problem related to non-contact monitoring a target individual to detect a fall and/or optionally detect a near fall. The target individual may be monitored within a closed environment, for example, a room in a house. The non-contact monitoring is performed remotely, without requiring the target individual to wear a device.

The ultrasound component(s) of the system, in comparison to other imaging modalities, consumes less energy, and/or the ultrasound signals may be processed using relatively fewer computational resources (e.g., processor utilization, processing time, and/or data storage requirements).

The spectrogram analysis extracts an indication of the movement of the target individual, which is processed to determine whether the target individual is falling. The features may be indicative of a near-fall, and/or whether the target individual is walking. The features may include multiple pieces of information, indicative of different aspects of the fall or near-fall, which are classified in combination by the classifier to compute the indication of the fall or near-fall. For example, the delay between the time of transmission and the time of receiving the reflection is indicative of the distance from the target individual to the ultrasound transceiver. A pattern of the delay (e.g., variation thereof) may be indicative of the fall or near-fall, for example, as the target individual moves relative to the floor while falling or near-falling. The frequency of the received reflection is indicative of the velocity of the target individual. A pattern in the frequency (e.g., variation thereof) of the received reflection may be indicative of the fall or near-fall, for example, as the target individual accelerates towards the floor during a fall, or changes velocity while moving around during a near-fall.

In a further implementation form of the first, second, and third aspects, the at least one processor is further configured for computing a complex signal centered around a zero frequency based on the reflection of the ultrasound transmission.

The complex signal provides additional data used for computation of the indication by the classifier, in addition to the frequency (e.g., frequency pattern) of the received reflection of the transmitted ultrasound. For example, the frequency of the carrier wave.

In a further implementation form of the first, second, and third aspects, the at least one processor is further configured for computing a spectral Doppler signature, wherein the plurality of features are extracted from the spectral Doppler signature.

In a further implementation form of the first, second, and third aspects, the at least one processor is further configured for and/or the method further comprises and/or the computer program product includes additional instructions for computing the complex signal by performing quadratic demodulation of the received reflection of the ultrasound transmission.

In a further implementation form of the first, second, and third aspects, the quadratic demodulation is performed by multiplication by a complex exponent having a carrier frequency corresponding to the difference between a transmission frequency of the ultrasound and a sampling frequency of the received reflection of the transmitted ultrasound.

Additional data associated with the carrier frequency may be used by the classifier for computation of the indication. For example, when the complex exponent has a frequency of 8 kHz, the complex exponent may be mathematically represented as exp($-i*2\pi f_c t$) where $f_c$ denotes the carrier frequency =8kHz and $i = \sqrt{-1}$ .

In a further implementation form of the first, second, and third aspects, the complex signal centered around zero Hz comprises an IQ quadratic demodulated signal.

The IQ quadratic demodulated signal provides additional data used by the classifier for computation of the indication, including the in-phase component of the received reflection and/or the quadrature component of the received reflection.

In a further implementation form of the first, second, and third aspects, the at least one processor is further configured for and/or the method further comprises and/or the computer program product includes additional instructions for decimating the received signal.

The decimation may reduce processing resource requirements (e.g., processor utilization, processing time, memory requirements) without necessarily and/or significantly reducing the accurate of the classifier to perform the computation of the indication.

In a further implementation form of the first, second, and third aspects, the plurality of features comprise one or more of: spectral features, temporal features, cepstral features, and any combination of the aforesaid.

In a further implementation form of the first, second, and third aspects, the at least one processor is further configured for and/or the method further comprises and/or the computer program product includes additional instructions to compute a short time Fourier transform (STFT) representation according to a predefined range of time of the received reflection of ultrasound, wherein the plurality of features are extracted from the STFT.

In a further implementation form of the first, second, and third aspects, the at least one processor is further configured for and/or the method further comprises and/or the computer program product includes additional instructions for dividing the received reflection into a plurality of time windows of a predefined size, computing the indication for each time window, and generate an overall indication of the fall or near-fall when a plurality of common indications are computed for respective time windows within a predefined time interval.

Identifying the fall or near fall is more accurate when multiple time windows are analyzed. The size of the time window may be selected to capture a fall or near-fall occurring within most or throughout the entirety of the time window, such that signals associated with the fall or near fall dominate the time window. Consecutive time windows may overlap to further increase the chance of one or more of the time windows entirely capturing the fall or near-fall. For example, time windows may be from about 1-64 milliseconds or other values. Time windows may partially overlap, for example, overlapping about 1-99%, or other ranges. If the time window is too large, an incorrect conclusion may be reached in which no fall or near-fall is detected (when a fall or near-fall has actually occurred) due to the dominating presence of signals not associated with a fall or near-fall in the time window. When the fall or near-fall is detected within multiple time windows within a range of time selected to capture the fall or near-fall, the accuracy of detection of the fall or near-fall is increased. For example, when two or more fall or near-fall indications are computed within a period of about 2-3 seconds (range of time selected to capture the whole or most of the fall or near-all event), the accuracy of detection of the fall or near-fall is increased.

In a further implementation form of the first, second, and third aspects, the at least one ultrasound transceiver is configured to generate the ultrasound transmission at a first predetermined frequency centered at about 40 kilohertz (kHz), within a bandwidth of about 37-43 kHz.

The relatively low frequency produces ultrasound with relatively large wavelengths, which are able to travel from the transceiver to the target individual located in proximity to the transceiver (e.g., about 1-5 meters), and for the reflection to return. The relatively low frequency is sufficient for imaging relatively large anatomical structures of the target individual for detection of the fall and/or near fall, for example, the body of the target individual, the torso, the arms, and/or the legs.

The relatively low frequency which transmits relatively large wavelengths, covers a relatively broad volume, and may be used to monitor the target individual within a room.

In a further implementation form of the first, second, and third aspects, the at least one processor is further configured for and/or the method further comprises and/or the computer program product includes additional instructions for sampling the received reflection of the ultrasound transmission at a predetermined sampling frequency that is higher than a predefined transmit frequency of the transmitted ultrasound and less than the Nyquist frequency of the transmit frequency.

The sampling frequency for the received ultrasound reflections may be lower than the Nyquist frequency of the transmitted ultrasound, since the bandwidth of the transmission frequency is limited to a predefined range. For example, for a transmission frequency of 40 kHz within a band of 37-43 kHz, the Nyquist frequency is 80 kHz (i.e., twice the transmission frequency), and the sampling frequency may be selected as 48000 samples per second.

In a further implementation form of the first, second, and third aspects, the at least one processor is further configured for and/or the method further comprises and/or the computer program product includes additional instructions for applying a band pass filter to the received reflection of the ultrasound transmission, wherein the band pass filter is selected according to a sampling frequency and a transmission frequency, to leave one real component of a replication created by the sampling of the reflection of the ultrasound transmission signal closest to zero.

Sampling the signal of the received reflection of the ultrasound transmission results in a replication located around the value of the frequency corresponding to the difference between the sampling frequency and the transmit frequency. For example, when the transmit frequency is 40 kHz and the sampling frequency is 48 kHz, the replication is located at about 8 kHz (i.e., 48 - 40). Another replication is created at the value of the frequency corresponding to the sum of the sampling frequency and the transmit frequency. For example, when the transmit frequency is 40 kHz and the sampling frequency is 48 kHz, the second replication frequency is located at about 88 kHz (i.e., 40 + 48). The frequency of the bandpass filter is selected to leave the real replication components closest to 0 Hz, for example, when the replication is expected to appear at about 8 kHz, the bandpass filter may be selected as 5-11 kHz.

In a further implementation form of the first, second, and third aspects, the at least one processor is further configured for and/or the method further comprises and/or the computer program product includes additional instructions for storing the indication of the near-fall and an associated timestamp in a memory, monitoring for at least one additional indication of near-fall computed within at least a predefined time interval of the timestamp, and generating an alert indicative of a plurality of near-fall events.

Near-fall events are monitored to detect multiple non-consecutive (i.e., occurring at different points in time, not related to a common near-fall event) near-fall events. Multiple near-fall events may be indicative of an impending fall. For example, based on an assumption that the multiple near-fall events are indicative of weakening of the target individual.

In a further implementation form of the first, second, and third aspects, the at least one processor is further configured for and/or the method further comprises and/or the computer program product includes additional instructions for analyzing the received reflection of the ultrasound transmission according to a set-of-rules, ignoring the received reflection when the set-of-rules is not met and processing the reflection to compute the plurality of features when the set-of-rules is met.

The received reflected signal may be ignored and/or excluded from further processing when the target individual is not present within the path of the transmitted ultrasound signals, and/or when the target individual is at rest and/or otherwise not moving. In such cases, insufficient energy may be received by the ultrasound transceiver. Ignoring and/or discarding the received reflected signal improves efficiency of the system, by reducing energy requirements and/or reducing processing resource requirements. The received reflected signals not need to be processed, since the processing is not expected to produce a result indicating fall or near-fall.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIG. 1 is a flowchart of a method of non-contact computation of an indication of a fall and/or near fall of a target individual from received reflected US signals, in accordance with some embodiments of the present invention;

FIG. 2 is a block diagram of components of a system for non-contact computation of an indication of a fall and/or near fall of a target individual based on received reflected US signals, in accordance with some embodiments of the present invention;

FIG. 3 is an exemplary implementation of a system for computation of an indication of a fall and/or a near fall of a target individual based on ultrasound, in accordance with some embodiments of the present invention; and

FIG. 4 is an exemplary implementation of a process of computing an indication of a fall and/or a near-fall of a target individual based on ultrasound, in accordance with some embodiments of the present invention.

DETAILED DESCRIPTION

The present invention, in some embodiments thereof, relates to fall detection and, more specifically, but not exclusively, to systems and methods for non-contact detection of a fall or near fall of a target individual.

An aspect of some embodiments of the present invention relates to systems, apparatus, methods, and/or code instructions for detection of a fall and/or near-fall of a monitored target individual based on non-contact ultrasound (US). One or more ultrasound transceivers generate ultrasound transmissions for monitoring the target individual. The received reflection of the ultrasound transmission is analyzed by a processors(s) performing a spectrogram analysis of the received reflection of the ultrasound transmission for computation of features. A trained statistical classifier(s) classifies the features into an indication of the fall and/or near fall of the target individual. An alert may be triggered in response to the indication of the fall and/or near fall, for example, a message is transmitted for presentation on a display of a client terminal.

Optionally, a complex signal centered around a zero frequency is computed based on the reflection of the ultrasound. The complex signal may be computed by performing quadratic demodulation of the received reflection of the ultrasound. The features are extracted from the complex signal. Alternatively or additionally, a distribution of frequencies (also referred to herein as *spectral Doppler signature*) is computed based on the reflection of the ultrasound. The features are extracted from the spectral Doppler signature.

The systems, apparatus, methods, and/or code instruction described herein address the technical problem related to non-contact monitoring of a target individual to detect a fall and/or optionally detect a near fall. The target individual may be monitored within a closed environment, for example, a room in a house. The non-contact monitoring is performed remotely, without requiring the target individual to wear a device.

It is noted that although the transmitted ultrasound is reflected off the target individual, the reflection is considered as non-contact herein.

The ultrasound component(s) of the system, in comparison to other imaging modalities, consumes less energy, and/or the ultrasound signals may be processed using relatively fewer computational resources (e.g., processor utilization, processing time, and/or data storage requirements).

The spectrogram analysis extracts an indication of the movement of the target individual, which is processed to determine whether the target individual is falling. The features may be indicative of a near-fall, and/or whether the target individual is walking. The features may include multiple pieces of information, indicative of different aspects of the fall or near-fall, which are classified in combination by the classifier to compute the indication of the fall or near-fall. For example, the delay between the time of transmission and the time of receiving the reflection is indicative of the distance from the target individual to the ultrasound transceiver. A pattern of the delay (e.g., variation thereof) may be indicative of the fall or near-fall, for example, as the target individual moves relative to the floor while falling or near-falling. The frequency of the received reflection is indicative of the velocity of the target individual. A pattern in the frequency (e.g., variation thereof) of the received reflection may be indicative of the fall or near-fall, for example, as the target individual accelerates towards the floor during a fall, or changes velocity while moving around during a near-fall.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

As used herein, the term *near-fall* means an intermediate state between stability (e.g., standing, walking, performing an activity such as changing, cooking, and accessing an item on a high shelf) and a fall, for example, losing balance which is regained without falling.

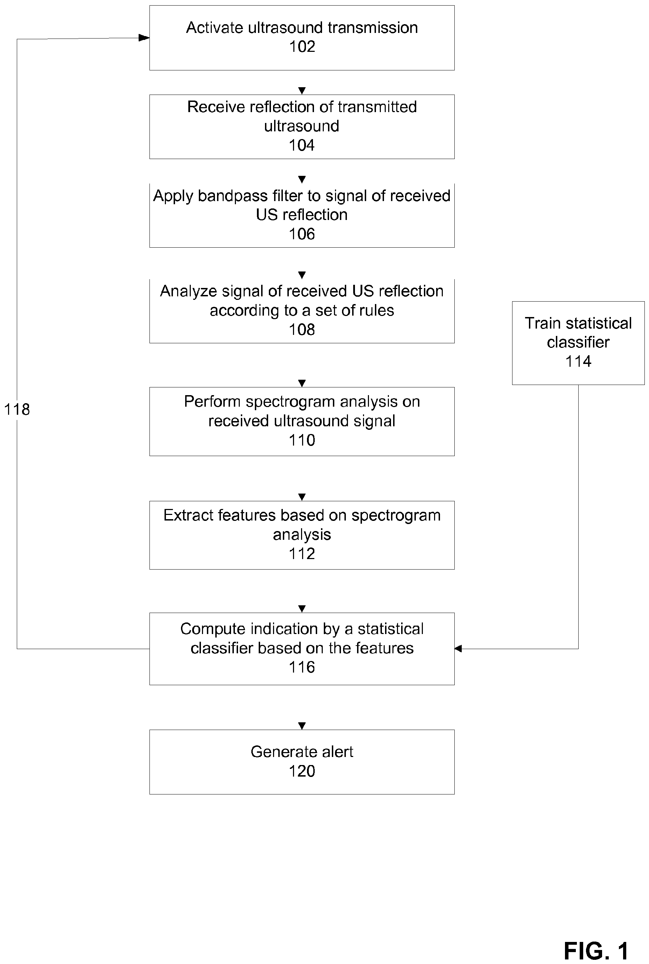

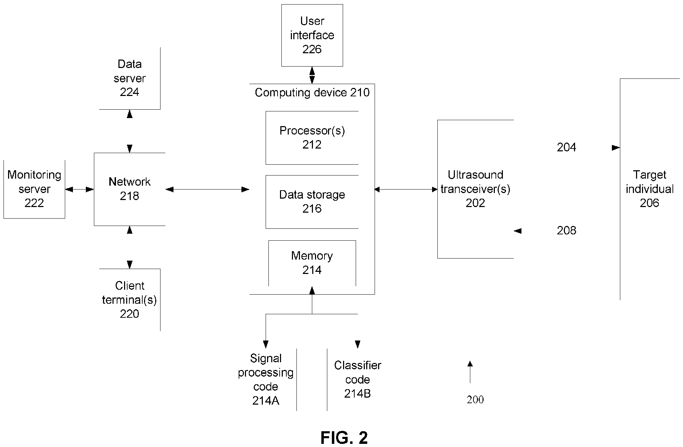

Reference is now made to FIG. 1, which is a flowchart of a method of non-contact computation of an indication of a fall and/or near fall of a target individual from received reflected US signals, in accordance with some embodiments of the present invention. Reference is also made to FIG. 2, which is a block diagram of components of a system 200 for non-contact computation of an indication of a fall and/or near fall of a target individual based on received reflected US signals, in accordance with some embodiments of the present invention. System 200 may implement the acts of the method described with reference to FIG. 1.

One or more ultrasound transceivers 202 transmit ultrasound energy 204 for monitoring of a target individual 206, and/or receive reflection 208 of the transmitted ultrasound energy 206 reflected off target individual 206. It is noted that different architectures may be implemented, for example, separate ultrasound devices for transmission and reception, or a single device capable of both transmission and reception. As used herein, the term *transceiver* is meant to cover the different implementations, and may include different physical devices for transmission and reception. One or more ultrasound transceivers 202 may be implemented, for example, by multiple devices located in different parts of a monitored room, and/or located in different rooms of a monitored apartment, and/or an array of multiple transceivers 202 arranged to cover a large room. Ultrasound transceiver(s) 202 may be implemented as ultrasound radar.

The transmission frequency of ultrasound transceiver 202 may be fixed according to the hardware implementation, and/or may be adjustable. An exemplary transmit frequency is centered at about 40 kilohertz (kHz), within a bandwidth of about 37-43 kHz. The relatively low frequency produces ultrasound with relatively large wavelengths, which are able to travel from the transceiver to the target individual located in proximity to the transceiver (e.g., about 1-5 meters), and for the reflection to return. The relatively low frequency is sufficient for imaging relatively large anatomical structures of the target individual for detection of the fall and/or near fall, for example, the body of the target individual, the torso, the arms, and/or the legs.

The relatively low frequency which transmits relatively large wavelengths, covers a relatively broad volume, and may be used to monitor the target individual within a room.

An exemplary implementation of ultrasound transmitter 202 may be found, for example, as model MA40S4S available from MURATA. An exemplary implementation of ultrasound receiver 202 may be found, for example, as model MA40S4R available from MURATA Manufacturing Co., at http://www(dot)murata(dot)com.

A computing device 210 is in electrical communication with US transceivers 202. Computing device 210 may activate US transceivers 202 to transmit ultrasound energy 204. US energy 204 is transmitted towards a target individual 206 being monitored for a fall or near fall. At least some of the transmitted signals are reflected off target individual 206 and received as reflected US 208 by US transceiver(s) 202. US transceiver(s) 202 may convert the received US 208 into a digital signal. Computing device 210 receives the digital signal from US transceiver(s) 202 and processes the received digital signal to compute the indication of the fall and/or near all of target individual 206. As used herein, the term *computing device* may sometimes be interchanged with the term *processor.*

Computing device 210 may receive the digital signal of the US reflections using one or more physical and/or virtual interface(s) 210, for example, a wire connection, a local bus connection, a wireless connection, other physical interface implementations, and/or virtual interfaces (e.g., software interface, application programming interface (API), software development kit (SDK)).

Computing device 210 may be implemented as, for example, a customized unit (e.g., hardware box which may be installed on a wall), a client terminal, a server, a computing cloud, a mobile device, a desktop computer, a thin client, a Smartphone, a Tablet computer, a laptop computer, a wearable computer, glasses computer, or a watch computer.

Computing device 210 may be integrated with US transceiver(s) 202, for example within a common housing. Alternatively or additionally, computing device 210 may be implemented as an independent and/or separate unit of US transceiver(s) 202, for example, within separate housings. For example, a single computing device 210 may be centrally installed within the home of the target individual. Multiple ultrasound transceivers 202 may be installed in geographical locations of the home that are considered as high risk for a fall, for example, a bathroom, a stairwell, and a kitchen. Ultrasound transceivers 202 may be installed throughout the living quarters of the target individual, to fully monitor the target individual for falls. Multiple ultrasound transceivers 202 may communicate with computing device 210 which may be centralized, for example, over a wireless network. In another implementation, where ultrasound transceiver(s) are integrated with computing device 210, multiple such integrated units may be installed throughout the home. The integrated units may communicate with each other, for example, over a mesh network, and/or via an internet of things (IoT) infrastructure.

In an exemplary architectural implementation, computing device 210 includes locally stored software that performs one or more of the acts described with reference to FIG. 1. In another exemplary architectural implementation, computing device 210 is remotely located, acting as one or more servers (e.g., network server, web server, a computing cloud) that provides centralized computational services to one or more components (e.g., local client terminals) that provide communication between US transceivers 202 and computing device 210 over a network.

Processor(s) 212 of computing device 210 may be implemented, for example, as a central processing unit(s) (CPU), a graphics processing unit(s) (GPU), field programmable gate array(s) (FPGA), digital signal processor(s) (DSP), and application specific integrated circuit(s) (ASIC). Processor(s) 212 may include one or more processors (homogenous or heterogeneous), which may be arranged for parallel processing, as clusters and/or as one or more multi core processing units.

A memory 214 stores code instructions implementable by processor(s) 212, for example, a random access memory (RAM), read-only memory (ROM), and/or a storage device, for example, non-volatile memory, magnetic media, semiconductor memory devices, hard drive, removable storage, and optical media (e.g., DVD, CD-ROM). Memory 214 may store signal processing code 214A that processes received US reflections 208, and/or may store trained classifier code 214B that computes the indication(s) from extracted features and/or may store other code, as described with reference to one or more acts of the method of FIG. 1. It is noted that one or more features of code 214A and/or code 214B may be implemented in hardware and/or firmware.

Computing device 210 may include a data storage device 216 for storing data, for example, a history of computed indications and associated timestamps. Data storage device 216 may be implemented as, for example, a memory, a local hard-drive, a removable storage unit, an optical disk, a storage device, and/or as a remote server and/or computing cloud (e.g., accessed using a network connection).

Computing device 204 may communicate over network 218 (or another communication channel, such as through a direct link (e.g., cable, wireless) and/or indirect link (e.g., via an intermediary computing unit such as a server, and/or via a storage device) with one or more of:

  * Client terminal(s) 220, for example, for transmission of an alert indicative of the fall and/or near-fall of the target individual. Client terminal(s) 220 may include, for example, a smartphone, a mobile device, a glasses computer, a tablet computer, a telephone, a desktop computer, a virtual machine, and a server. The alert may be presented on a display of client terminal 220, and/or an audio message may be played (e.g., on a speaker of a phone).
  * Monitoring server 222, for example, of a health organization that monitors senior citizens at home.
  * Data server 224, which may provide updated versions of the trained statistical classifier (which may be locally downloaded to computing device(s) 210 and stored as code 214B), and/or which may receive additional training data from computing device(s) 210 for updating of the classifier.

Exemplary networks 218 may include but is not limited to: the internet, a local area network, a virtual network, a wireless network, a cellular network, a point to point network, a short distance wireless connection, and a cable connection.

Computing device 210 or client terminal 226 can include and/or be in communication with a user interface 226 that includes a mechanism for a user to enter data and/or view presented data (e.g., the computed indication). Exemplary user interfaces 226 include, for example, one or more of: a touchscreen, a display, a keyboard, a mouse, and voice activated software using speakers and microphone.

Referring now to FIG. 1, at 102, ultrasound transceiver(s) 202 is activated for generating ultrasound transmission 204. Ultrasound transceiver 202 may be activated based on instructions received from computing device 210 and/or from another component, for example, a physical on/off button.

Ultrasound transceiver 202 may operate in continuous on mode, transmitting ultrasound energy 204 whether or not the target individual is present in the room or not. Alternatively, ultrasound transceiver 202 is activated according to a trigger, for example, a motion sensor that detects when the target patient entered the room.

Ultrasound transceiver 202 may transmit a continuous signal, optionally a sinus signal, for example, when separate US transmitters and US receivers are implemented. Alternatively, ultrasound transceiver 202 transmits in pulses separated by no-transmission intermission periods, for example, to sense the reflection of the transmission during the intermission periods.

At 104, ultrasound transceiver(s) 202 receives reflection 208 of the ultrasound transmission.

The received signal may be an analog signal, which is sampled by hardware and/or code components of transceiver(s) 202, and/or computing device 210 and/or other circuitry.

Optionally, the predetermined sampling frequency is higher than the predefined transmit frequency of the transmitted ultrasound and less than the Nyquist frequency of the transmit frequency. The sampling frequency for the received ultrasound reflections may be lower than the Nyquist frequency of the transmitted ultrasound, since the bandwidth of the transmission frequency is limited to a predefined range. For example, for a transmission frequency of 40 kHz within a band of 37-43 kHz, the Nyquist frequency is 80 kHz (i.e., twice the transmission frequency), and the sampling frequency may be selected as 48000 samples per second.

At 106, optionally, a band pass filter is applied to the signal of the received reflection of the ultrasound transmission. The band pass filter may be applied to the raw received signal (e.g., analog signal prior to sampling) and/or to the sampled signal. The band pass filter may be implemented in hardware and/or code. Sampling the signal of the received reflection of the ultrasound transmission results in a replication located around the value of the frequency corresponding to the difference between the sampling frequency and the transmit frequency. The band pass filter is selected according to the sampling frequency and the US transmission frequency, to leave one real component of a replication created by the sampling of the reflection of the ultrasound transmission signal closest to zero. For example, when the transmit frequency is 40 kHz and the sampling frequency is 48 kHz, the replication is located at about 8 kHz (i.e., 48 - 40). The replication component closest to zero frequency is the 8 kHz component, which is retained. Another replication is created at the value of the frequency corresponding to the sum of the sampling frequency and the transmit frequency. For example, when the transmit frequency is 40 kHz and the sampling frequency is 48 kHz, the second replication frequency is located at about 88 kHz (i.e., 40 + 48). The frequency of the band pass filter is selected to leave the real replication components closest to 0 Hz, for example, when the replication is expected to appear at about 8 kHz, the bandpass filter may be selected as 5-11 kHz.

At 108, optionally, the received reflection 208 of the ultrasound transmission and/or sampled signal thereof is analyzed according to a set of rules. When the set of rules is not met, the received signal of the US reflection is ignored and/or discarded, without performing the classification to compute the indication. Alternatively, when the set of rules is met, the signal of the US reflection is processed as described herein to compute the features and perform the classification for computation of the indication. The set of rules may define initial signal processing, for example, to determine whether the reflected signal has been reflected off the target individual or not (i.e., whether the target individual is being monitored or not). Reflection of the transmitted US off the target individual may be detected, for example, by identifying a statistically significant change relative to a baseline reflected US signal. The baseline signal may represent the reflected US signal when the target individual is not present within the path of the transmitted US, for example, the US reflecting off walls and furniture in the room. Since nothing is expected to be moving in the room when the target individual is absent, the baseline reflected US signal is assumed to be constant (e.g., within a tolerance representing statistical insignificant variation). The appearance of the target individual causes a statistically significant difference in the newly received US signal in comparison to the baseline signal. The received reflected signal may be ignored and/or excluded from further processing when the target individual is not present within the path of the transmitted ultrasound signals, and/or when the target individual is at rest and/or otherwise not moving. In such cases, insufficient energy may be received by the ultrasound transceiver. Ignoring and/or discarding the received reflected signal improves efficiency of the system, by reducing energy requirements and/or reducing processing resource requirements. The received reflected signals do not need to be processed, since the processing is not expected to produce a result indicating fall or near-fall.

At 110, a spectrogram analysis of the reflection 208 of the ultrasound transmission is performed. The spectrogram analysis may be performed by code 214A executed by processor(s) 212.

Optionally, the spectrogram analysis includes computing a complex signal centered around a zero frequency, based on the reflection of the ultrasound transmission. The complex signal provides additional data used for computation of the indication by the classifier, in addition to the frequency (e.g., frequency pattern) of the received reflection of the transmitted ultrasound. For example, the frequency of the carrier wave.

Optionally, the complex signal is computed by performing quadratic demodulation of the received reflection of the ultrasound transmission. The quadratic demodulation is performed by multiplication by a complex exponent having a carrier frequency corresponding to the difference between a transmission frequency of the ultrasound and a sampling frequency of the received reflection of the transmitted ultrasound. Additional data associated with the carrier frequency may be used by the classifier for computation of the indication. For example, when the complex exponent has a frequency of 8 kHz, the complex exponent may be mathematically represented as $\exp(-i*2\pi f_c t)$ where $f_c$ denotes the carrier frequency =8kHz and $i = \sqrt{-1}$ .

The complex signal centered around zero Hz may be implemented as an IQ quadratic demodulated signal. The IQ quadratic demodulated signal provides additional data used by the classifier for computation of the indication, including the in-phase component of the received reflection and/or the quadrature component of the received reflection.

Alternatively or additionally, the spectrogram analysis includes computing a spectral Doppler signature.

Optionally, the received signal may be decimated. The decimation may be performed for the sampled signal, and/or for the complex signal. Decimation may be performed, for example, by applying a multi-rate filter. For example, every eighth sample (for 48 kHz sampling, or other sampling frequencies may be used) may be stored after applying a lowpass filter, decreasing the sampling rate to 6 kHz from 48 kHz. The decimation may reduce processing resource requirements (e.g., processor utilization, processing time, memory requirements) without necessarily and/or significantly reducing the accuracy of the classifier to perform the computation of the indication.

Alternatively or additionally, a short time Fourier transform (STFT) representation is computed according to a predefined range of time of the received reflection of ultrasound.

At 112, multiple features are extracted based on the spectrogram analysis. The features may be extracted by code 214A executed by processor(s) 212.

The features may be extracted, for example, from one or more of: the complex signal(s), the IQ quadratic demodulated signal, the spectral Doppler signature, the STFT, and/or combinations of the aforementioned.

Exemplary features include: spectral features, temporal features, cepstral features, and any combination of the aforesaid. Features may be, for example, automatically learned by a neural network, hand crafted features, and/or automatically generated and/or automatically selected features which were generated and/or selected as part of the classifier training process.

Exemplary features may be automatically identified by a neural network implementation of the classifier. Features may be automatically extracted by the neural network as part of the classification process described with reference to act 116. For example, the neural network receives the STFT and/or other spectrogram representation and automatically extracts the learned features as part of the classification process.

At 114, the statistical classifier is trained. The training of the statistical classifier may be performed centrally for a general population of patients. The trained classifier may be distributed for local classification. The statistical classifier may be updated based on additional learning performed in the field based on real-life cases.

The statistical classifier is trained based on a training set of data that includes features and an associated defined indication also referred to as label, for example, a metadata tag associated with the features and/or a data entry stored in a database associated with the features. The indication may be manually labeled, for an example, by an observer that collects the received US measurements for feature extraction for the training set. The features are computed from received reflections of ultrasound within a respective predefined range of time, computed as described herein.

Alternatively or additionally, the statistical classifier is trained with the output of the spectrogram analysis or portion thereof, for example, STFT and/or complex signals, computed for each of multiple time windows. Each time window is associated with a defined indication (e.g., manually created metadata tag indicative of a classification category). The statistical classifier may automatically learn which features to extract, for example, when implemented as a neural network. The learned features may be stored and implemented with reference to act 112, and/or with reference to 116 when the features are automatically extracted by the neural network as part of the classification process. For example, the neural network receives the STFT and/or other spectrogram representation and automatically extracts features as part of the classification process.

Exemplary classifiers include but are not limited to support vector machine (SVM), neural network, logistic regression, decision trees, k-nearest neighbor, or any combination of the aforesaid.

It is noted that act 114 may be executed prior to act 102. The training of the classifier described with reference to act 114 may be executed by computing device 210, and/or by a remote computing (e.g., server) and obtained over network 218.

At 116, an indication is computed by a statistical classifier (e.g. classifier 214B executed by processor(s) 212) classifying the extracted features.

The indication may be implemented as a classification category of the classifier. The classifier may be a single category classifier, a binary classifier, or a classifier into multiple categories. The single category may include indication of a fall. In another implementation of the single category classifier, the single category includes an indication of a near-fall. The binary classifier may be implemented as classifying into an indication of a fall or a near-fall. The multiple category classifier may be implemented as classifying into an indication of a fall, a near-fall, walking motion of the target individual, and/or other categories.

Alternatively or additionally, the classifier outputs a probability associated with the indication. The probability indicates the likelihood that the target individual has fallen or has almost fallen. A threshold may be associated with the probability to determine whether the indication is generated or not. The threshold may be manually defined by a user, and/or automatically computed by code (e.g., by the classification training process). For example, the threshold may be set at 75%, where a probability of a fall or near fall about 75% triggers the indication of the fall or near-fall, and no indication is generated when the probability is below 75%.

At 118, one or more acts described with reference to 102-112 and 116 are iterated (it is noted that training of the classifier described with reference to act 114 is excluded from the iteration). Each iteration may process another signal of received US reflection. The signals may be spaced apart in time, or continuous. Blocks 102-112 and 116 may be executed iteratively for each new portion of received US reflection. Alternatively, the US reflection is received at 104, and divided into multiple time windows, with each time window being processed as described with reference to acts 106-112 and 116. For both cases, the received reflection is divided into time windows of a predefined size. The indication is computed for each time window. An overall indication of the fall or near-fall is computed when one or more (which may be predefined or dynamically determined) of common indications are computed for respective time windows within a predefined time interval.

Identifying the fall or near fall is more accurate when multiple time windows are analyzed. The size of the time window may be selected to capture a fall or near-fall occurring within most or throughout the entirety of the time window, such that signals associated with the fall or near fall dominate the time window. Consecutive time windows may overlap to further increase the chance of one or more of the time windows capturing the fall or near-fall. For example, time windows may be from about 1-64 milliseconds or other values. Time windows may partially overlap, for example, overlapping about 1-99%, or other ranges. If the time window is too large, an incorrect conclusion may be reached in which no fall or near-fall is detected (when a fall or near-fall has actually occurred) due to the dominating presence of signals not associated with a fall or near-fall in the time window. When the fall or near-fall is detected within multiple time windows within a range of time selected to capture the fall or near-fall, the accuracy of detection of the fall or near-fall is increased. For example, when two or more fall or near-fall indications are computed within a period of about 2-3 seconds (range of time selected to capture the whole or most of the fall or near-all event), the accuracy of detection of the fall or near-fall is increased.

Optionally, the indication of the near-fall and an associated timestamp is stored, for example, in a memory (e.g., data storage 216). Monitoring is performed for one or more (e.g., for a predefined number and/or a dynamically determined number of) additional indications of near-fall computed within at least a predefined time interval of the timestamp. The predefined time interval may be selected to distinguish between a pattern of near-falls which are indicative of an impending fall (and/or greater risk of a fall), and independent near-fall events which are not linked to one another and not indicative of a greater risk of falling, for example, a week, 2 weeks, and a month. An alert indicative of the multiple detect near-fall events is generated (as described with reference to act 120). Near-fall events are monitored to detect multiple non-consecutive (i.e., occurring at different points in time, not related to a common near-fall event) near-fall events. Multiple near-fall events may be indicative of an impending fall. For example, based on an assumption that the multiple near-fall events are indicative of weakening of the target individual.

For example, a first indication of a near-fall may be stored and additional monitoring of the target individual is performed. When a second non-consecutive indication of near-fall is computed within a predetermined period of time, for example, within a week, a message is transmitted to the monitoring server and/or client terminal. The message indicates that the target individual is at greater risk of falling, and/or that is it advisable to get help and/or exercise extra care to avoid a fall.

At 120, a message of the indication of the fall and/or the near fall is generated. The message may be, for example, a pop-up box that is pushed onto a display over an existing presentation, a message that appears within a graphical user interface (GUI) designed for monitoring of the target individual, an email, a text message, an audio message played over a speaker (e.g., phone call), and a flashing light. The message may be locally presented within user interface 226 of computing device 210 (e.g., display, speaker), and/or transmitted over network 218 to client terminal(s) 220 (e.g., of healthcare workers and/or other users caring for the target individual) and/or to monitoring server 222.

Upon receiving the message indicating that the target person has fallen or almost fallen, monitoring server 222 and/or client terminal(s) 220 may attempt to contact the target individual (e.g., a call to the phone of the target individual), and/or dispatch someone to check on the target individual, and/or the like.

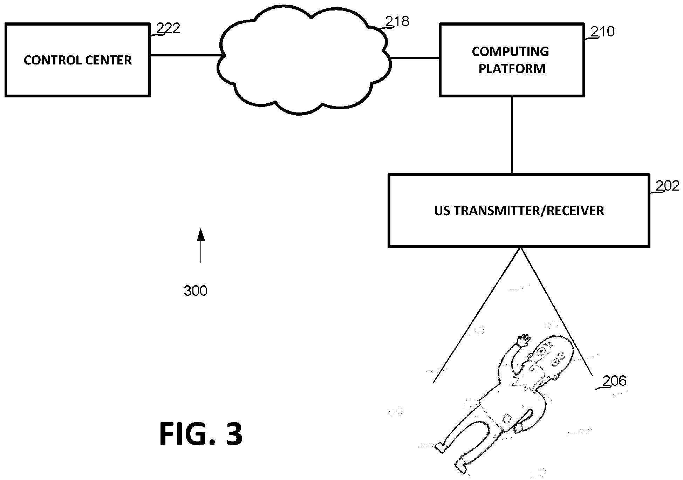

Reference is now made to FIG. 3, which is an exemplary implementation of a system 300 for computation of an indication of a fall and/or a near fall of a target individual 206 based on ultrasound, in accordance with some embodiments of the present invention. Components described with reference to system 300 of FIG. 3 correspond to components described with reference to system 200 of FIG. 2. US transmitter and/or receiver 202 transmits ultrasound energy for monitoring target individual 206, and receives reflections of the transmitted US energy. The received signals are processed by computing platform 210 as described herein, to compute an indication of a fall and/or near-fall. When the indication of the fall and/or near-fall is detected, a message is transmitted over network 218 to a control center 222 (e.g., monitoring server 222). Control center 222 may attempt to contact target individual 206 (e.g., phone call, text message) and/or dispatch someone to check on target individual 206.

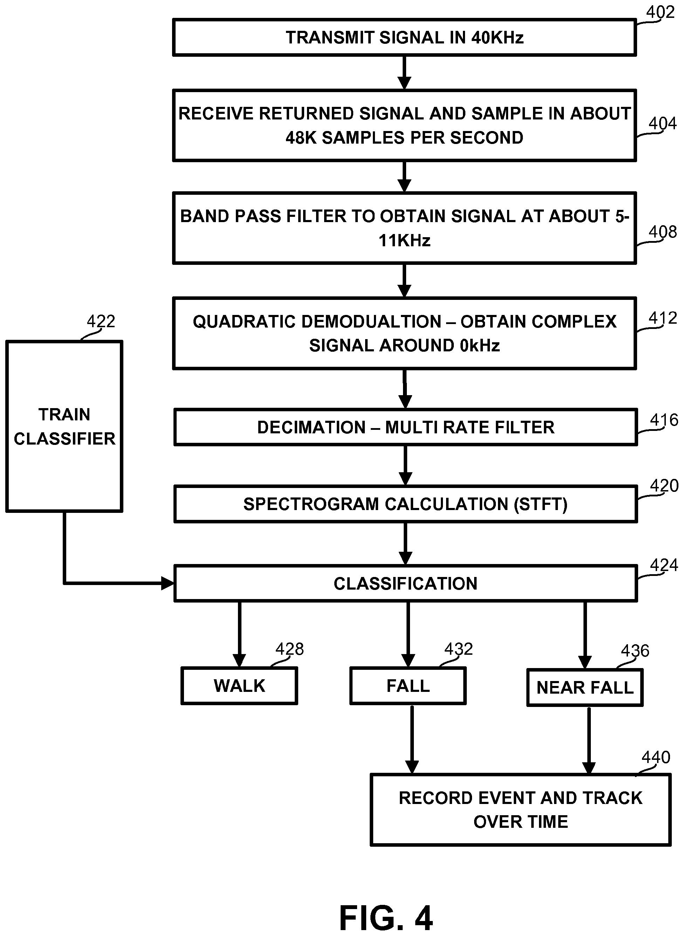

Reference is now made to FIG. 4, which is an exemplary implementation of a process of computing an indication of a fall and/or a near-fall of a target individual based on ultrasound, in accordance with some embodiments of the present invention. Features described with reference to the process of FIG. 4 may correspond to, and/or be integrated with features described with reference to the method of FIG. 1. The features of the process described with reference to FIG. 4 may be executed by components of system 200 described with reference to FIG. 2, and/or components of system 300 described with reference to FIG. 3.

At 402, an ultrasound signal is transmitted, for example, at 40 kHz. For example, as described with reference to act 102 of FIG. 1.

At 404, the reflection of the transmitted US signal is received. The received signal is sampled, for example, at 48000 samples per second. For example, as described with reference to act 104 of FIG. 1.

At 408, a bandpass filter is applied to the sampled signal. The bandpass filter is designed to leave one real component of a replication created by the sampling of the reflection of the ultrasound transmission signal closest to zero. The bandpass filter is designed, for example, to extract the signals within the band of 5-11 kHz, around the replication at 8 kHz. For example, as described with reference to act 106 of FIG. 1.

At 412, a quadratic demodulation of the filtered signal is computed to obtain a complex signal(s) centered around 0 kHz. For example, as described with reference to act 110 of FIG. 1.

At 416, the complex signal may be decimated, for example, by applying a multi-rate filter. For example, as described with reference to act 110 of FIG. 1.

At 420, features are extracted from the complex signal, optionally from a computed STFT representation. The features may be automatically learned features learned by a neural network, hand crafted features, and/or automatically generated and/or automatically selected features. For example, as described with reference to act 112 of FIG. 1.

At 422, a classifier is trained. For example, as described with reference to act 114 of FIG. 1.

At 424, the classifier performs a classification of the extracted features. For example, as described with reference to act 116 of FIG. 1.

At 428, an indication of walking is computed. The indication of walking denotes a state in which the target user is not falling or in a state of near-fall. The walking indication may be interchanged and/or in addition to other states, for example, stability state, resting state, and climbing state. Alternatively, at 432, an indication of falling is computed. Alternatively, at 436, an indication of near-fall is computed. For example, as described with reference to act 116 of FIG. 1.

At 440, when the indication of fall or near-fall is computed, the indication is recorded and tracked over time. Another fall or near-fall event is monitored, to determine whether the target individual is at increased risk of falling. For example, as described with reference to act 118 of FIG. 1. An alert is generated accordingly, for example, as described with reference to act 120 of FIG. 1.

The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

It is expected that during the life of a patent maturing from this application many relevant ultrasound transceivers will be developed and the scope of the term ultrasound transceiver is intended to include all such new technologies a priori.

As used herein the term "about" refers to ± 10 %.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

It will be understood that the invention has been described above purely by way of example, and modifications of detail can be made within the scope of the invention.

Each feature disclosed in the description, and (where appropriate) the claims and drawings may be provided independently or in any appropriate combination.

Reference numerals appearing in the claims are by way of illustration only and shall have no limiting effect on the scope of the claims.

**Claims**

1.  A system (200) for non-contact computation of an indication of at least one of a fall and a near-fall of a target individual (206) based on ultrasound transmission, comprising:

    at least one ultrasound transceiver (202) configured for generating an ultrasound transmission (204) and receiving a reflection (208) of the ultrasound transmission; and
    at least one processor (212) in communication with the ultrasound transceiver, the at least one processor configured for:

    activating (102) the at least one ultrasound transceiver for generating the ultrasound transmission, and for receiving (104) the reflection of the ultrasound transmission;
    performing (110) a spectrogram analysis of the received reflection of the ultrasound transmission to compute a plurality of features; and
    computing (116) an indication of at least one of: a fall of a target individual, and a near-fall of the target individual, by a statistical classifier classifying the plurality of features.

2.  The system (200) according to claim 1, wherein the at least one processor (212) is further configured for computing a complex signal centered around a zero frequency based on the reflection (208) of the ultrasound transmission (204).

3.  The system (200) according to claim 1 or claim 2, wherein the at least one processor (212) is further configured for computing a spectral Doppler signature, wherein the plurality of features are extracted from the spectral Doppler signature.

4.  The system (200) according to claim 2 or claim 3, wherein the at least one processor (212) is further configured for computing the or a complex signal by performing quadratic demodulation of the received reflection (208) of the ultrasound transmission (204).

5.  The system (200) according to claim 4, wherein the quadratic demodulation is performed by multiplication by a complex exponent having a carrier frequency corresponding to the difference between a transmission frequency of the ultrasound and a sampling frequency of the received reflection (208) of the transmitted ultrasound (204).

6.  The system (200) according to any one of claims 2-5, wherein the or a complex signal centered around zero Hz comprises an IQ quadratic demodulated signal.

7.  The system (200) according to any of the preceding claims, wherein the processor (212) is further configured to decimate the received signal (208).

8.  The system (200) according to any of the preceding claims, wherein the plurality of features comprise one or more of: spectral features, temporal features, cepstral features, and any combination of the aforesaid.

9.  The system (200) according to any of the preceding claims, wherein the processor (212) is further configured to compute a short time Fourier transform (STFT) representation according to a predefined range of time of the received reflection (208) of ultrasound, wherein the plurality of features are extracted from the STFT.

10. The system (200) according to any of the preceding claims, wherein the processor (212) is further configured for dividing the received reflection into a plurality of time windows of a predefined size, computing the indication for each time window, and generate an overall indication of the fall or near-fall when a plurality of common indications are computed for respective time windows within a predefined time interval.

11. The system (200) according to any of the preceding claims, wherein the at least one ultrasound transceiver (202) is configured to generate the ultrasound transmission (204) at a first predetermined frequency centered at about 40 kilohertz (kHz), within a bandwidth of about 37-43 kHz.

12. The system (200) according to any of the preceding claims, wherein the at least one processor (212) is further configured for sampling the received reflection (208) of the ultrasound transmission (204) at a predetermined sampling frequency that is higher than a predefined transmit frequency of the transmitted ultrasound and less than the Nyquist frequency of the transmit frequency.

13. The system (200) according to any of the preceding claims, wherein the at least one processor (212) is further configured for applying a band pass filter to the received reflection (208) of the ultrasound transmission (204), wherein the band pass filter is selected according to a sampling frequency and a transmission frequency, to leave one real component of a replication created by the sampling of the reflection of the ultrasound transmission signal closest to zero.

14. The system (200) according to any of the preceding claims, wherein the processor (212) is further configured for storing the indication of the near-fall and an associated timestamp in a memory, monitoring for at least one additional indication of near-fall computed within at least a predefined time interval of the timestamp, and generating an alert indicative of a plurality of near-fall events.

15. The system (200) according to any of the preceding claims, wherein the processor (212) is further configured for analyzing the received reflection (208) of the ultrasound transmission (204) according to a set-of-rules, ignoring the received reflection when the set-of-rules is not met and processing the reflection to compute the plurality of features when the set-of-rules is met.

Activate ultrasound transmission
102

▼

Receive reflection of transmitted ultrasound
104

▼

Apply bandpass filter to signal of received US reflection
106

▼

Analyze signal of received US reflection according to a set of rules
108

▼

Perform spectrogram analysis on received ultrasound signal
110

▼

Extract features based on spectrogram analysis
112

▼

Compute indication by a statistical classifier based on the features
116

▼

Generate alert
120

Train statistical classifier
114

118

FIG. 1

**FIG. 2**

FIG. 3

```
                                                                    402
       ┌─────────────────────────────────────────────────┐
       │         TRANSMIT SIGNAL IN 40KHz                 │
       └─────────────────────────────────────────────────┘
                            │
                            ▼
                                                                    404
       ┌─────────────────────────────────────────────────┐
       │  RECEIVE RETURNED SIGNAL AND SAMPLE IN ABOUT      │
       │          48K SAMPLES PER SECOND                   │
       └─────────────────────────────────────────────────┘
                            │
                            ▼
                                                                    408
       ┌─────────────────────────────────────────────────┐
       │ BAND PASS FILTER TO OBTAIN SIGNAL AT ABOUT 5-     │
       │                  11KHz                            │
       └─────────────────────────────────────────────────┘
                            │
                            ▼
                                                                    412
       ┌─────────────────────────────────────────────────┐
 422   │  QUADRATIC DEMODUALTION – OBTAIN COMPLEX          │
┌──────┐│         SIGNAL AROUND 0kHz                        │
│TRAIN ││└─────────────────────────────────────────────────┘
│CLASS-││                  │
│IFIER ││                  ▼
│      ││                                                          416
└──────┘│ ┌─────────────────────────────────────────────────┐
        │ │      DECIMATION – MULTI RATE FILTER              │
        │ └─────────────────────────────────────────────────┘
        │                  │
        │                  ▼
        │                                                          420
        │ ┌─────────────────────────────────────────────────┐
        │ │    SPECTROGRAM CALCULATION (STFT)                │
        │ └─────────────────────────────────────────────────┘
        │                  │
        │                  ▼
        │                                                          424
        └─►┌─────────────────────────────────────────────────┐
           │              CLASSIFICATION                       │
           └─────────────────────────────────────────────────┘
              │              │              │
              ▼              ▼              ▼
            428            432            436
         ┌───────┐     ┌───────┐     ┌──────────┐
         │ WALK  │     │ FALL  │     │NEAR FALL │
         └───────┘     └───────┘     └──────────┘
                          │              │
                          ▼              ▼
                                              440
                    ┌─────────────────────────────┐
                    │  RECORD EVENT AND TRACK      │
                    │         OVER TIME            │
                    └─────────────────────────────┘
```

# FIG. 4

Europäisches
Patentamt

European
Patent Office

Office européen
des brevets

# EUROPEAN SEARCH REPORT

Application Number

EP 17 18 9288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ARVINDKUMAR CHAVDA ET AL: "Non-intrusive user-oriented interactive fall-detection system for seniors", 2015 IEEE CANADA INTERNATIONAL HUMANITARIAN TECHNOLOGY CONFERENCE (IHTC2015), IEEE, 31 May 2015 (2015-05-31), pages 1-4, XP033214901, DOI: 10.1109/IHTC.2015.7238071 ISBN: 978-1-4799-8961-4 [retrieved on 2015-09-02] | 1-9,11, 13,14 | INV. A61B5/11 G08B21/04 G16H40/67 A61B8/08 |
| A | * the whole document * ----- | 10,12,15 | |
| A | DURA-BERNAL SALVADOR ET AL: "Human Action Categorization Using Ultrasound Micro-Doppler Signatures", 16 November 2011 (2011-11-16), NETWORK AND PARALLEL COMPUTING; [LECTURE NOTES IN COMPUTER SCIENCE; LECT.NOTES COMPUTER], SPRINGER INTERNATIONAL PUBLISHING, CHAM, PAGE(S) 18 - 28, XP047436662, ISSN: 0302-9743 ISBN: 978-3-642-01969-2 * the whole document * ----- | 1-15 | |
| A | US 7 916 066 B1 (OSTERWEIL JOSEF [US]) 29 March 2011 (2011-03-29) * the whole document * ----- | 1-15 | TECHNICAL FIELDS SEARCHED (IPC)  A61B G08B G16H |

The present search report has been drawn up for all claims

| Place of search | Date of completion of the search | Examiner |
|---|---|---|
| Munich | 23 January 2018 | Koprinarov, Ivaylo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 18 9288

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2018

| Patent document cited in search report | | Publication date | Patent family member(s) | | Publication date |
|---|---|---|---|---|---|
| US 7916066 | B1 | 29-03-2011 | US | 7916066 B1 | 29-03-2011 |
| | | | US | 8068051 B1 | 29-11-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62383443 B